(19) Europäisches Patentamt
European Patent Office
Office européen des brevets

(11) **EP 2 010 200 B1**

(12) **FASCICULE DE BREVET EUROPEEN**

(45) Date de publication et mention de la délivrance du brevet:
**05.07.2017 Bulletin 2017/27**

(21) Numéro de dépôt: **07728332.3**

(22) Date de dépôt: **20.04.2007**

(51) Int Cl.:
*C12P 19/26* (2006.01)     *A61K 38/00* (2006.01)

(86) Numéro de dépôt international:
**PCT/EP2007/053873**

(87) Numéro de publication internationale:
**WO 2007/122187 (01.11.2007 Gazette 2007/44)**

(54) **UTILISATION DE POLYSACCHARIDES D'ORIGINE FONGIQUE COMME COMPOSITION PHARMACEUTIQUE OU COMPLEMENTS ALIMENTAIRES**

ANWENDUNG VON POLYSACCHARIDEN AUS FUNGI ALS PHARMAZEUTISCHEN ZUSAMMENSETZUNG ODER ALS NAHRUNGSMITTELERGÄNZUNG

USE OF FUNGAL POLYSACCHARIDES AS PHARMACEUTICAL COMPOSITION OR FOOD COMPLEMENTS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **21.04.2006 FR 0651415**

(43) Date de publication de la demande:
**07.01.2009 Bulletin 2009/02**

(73) Titulaire: **Kitozyme S.A.**
**4040 Herstal (BE)**

(72) Inventeurs:
• **TEISSEDRE, Pierre-Louis**
**F-33600 Pessac (FR)**
• **BORNET, Aurélie**
**F-74380 Lucinges (FR)**
• **GAUTIER, Sandrine**
**B-4000 Liege (BE)**
• **BRUYERE, Jean-Michel**
**B-4000 LIEGE (BE)**
• **ROUANET, Jean-Max**
**F-34980 Saint Gely du Fesc (FR)**

(74) Mandataire: **Lavoix**
**2, place d'Estienne d'Orves**
**75441 Paris Cedex 09 (FR)**

(56) Documents cités:
**WO-A-03/057233     WO-A-03/068824**
**WO-A2-01/35944     CZ-A3- 9 200 664**

• **KOGAN G ET AL: "Fungal chitin-glucan derivatives exert protective or damaging activity on plasmid DNA" CARBOHYDRATE RESEARCH, ELSEVIER SCIENTIFIC PUBLISHING COMPANY. AMSTERDAM, NL, vol. 338, no. 9, 22 avril 2003 (2003-04-22), pages 931-935, XP004418228 ISSN: 0008-6215**
• **DATABASE WPI Week 199421 Derwent Publications Ltd., London, GB; AN 1994-168171 XP002411985 & CZ 9 200 664 A3 (MIKROBIOLOGICKY USTAV AVCR) 13 avril 1994 (1994-04-13)**

Remarques:
Le dossier contient des informations techniques présentées postérieurement au dépôt de la demande et ne figurant pas dans le présent fascicule.

## Description

L'invention concerne l'utilisation de polysaccharides d'origine fongique comme composition pharmaceutique ou compléments alimentaires pour la santé humaine et animale.

### Etat de l'art

Il est connu que les champignons, aussi bien microscopiques que comestibles, présentent des propriétés bénéfiques pour la santé, telles que hypoglycémique, hypocholestérolémiante, antioxydante, immunostimulante. Les champignons contiennent des composés digestibles ayant des propriétés importantes sur la santé du tractus gastro-intestinal et du corps en général, définis comme étant des fibres alimentaires de type polysaccharide. Ils présentent la propriété d'agir sur la circulation de glucose en abaissant la concentration sanguine en insuline, augmentent la viscosité des aliments dans le petit intestin et ralentissent l'absorption des glucides. Ils possèdent donc la capacité de réguler le métabolisme des sucres. Ils jouent également un rôle dans la régulation de la tension artérielle. Les champignons qui contiennent des quantités importantes de fibres ont un effet bénéfique sur la baisse de la concentration en cholestérol total, et du HDL-cholesterol au niveau sanguin, comme montré par une étude de Fukushima et al. (2000) sur des fibres de champignon *Agaricus bisporus* (Fukushima M, Nakano M, Morii Y, Ohashi T, Fujiwara Y & Sonoyama K (2000) J. Nutr. 130:2151). Ils jouent donc un rôle indirect sur la prévention de l'hypertension, des maladies cardiovasculaires, et plus largement sur l'obésité et le syndrome métabolique.

Très peu d'aliments exercent des propriétés immunostimulantes. Pour la plupart, les produits alimentaires induisent plutôt une immunodépression. Des champignons comme le shii-také (*Lentinus edodes*), le maïtaké (*Grifola frondosa*), le reishi (*Ganoderma lucidum*) ou l'ABM (*Agaricus blazei murill*) sont des aliments dits immunostimulants. La fonction immunostimulante est largement attribuée à la présence des polysaccharides de type beta-glucane présents dans leurs parois cellulaires (Lull C, Wichers HJ & Savelkoul HFJ (2005) Antiinflammatory and immunomodulating properties of fungal metabolites. Mediators Inflammation 2:63).

Certaines fibres végétales sont recommandées pour prévenir, inhiber ou traiter l'obésité et les maladies associées à l'obésité, comme par exemple les oligofructoses dérivés de l'inuline de chicorée, ou la laminarine, un beta-glucane issu des algues. Les oligofructoses agissent en fermentant au niveau du colon, libérant ainsi des composés capables de supprimer la teneur plasmatique en ghreline, une hormone qui stimule habituellement l'appétit. Indirectement, en augmentant la satiété et en réduisant la prise d'aliments, on observe un effet sur le taux de cholestérol et l'athérosclérose, parmi les nombreux effets associés au syndrome métabolique et aux maladies cardiovasculaires.

Le chitosane, un polysaccharide traditionnellement issu de la chitine des carapaces de crustacés et considéré comme une fibre, est également connu pour exercer une action hypolipidémiante et hypocholestérolémiante. Ces effets sont attribués à un mécanisme d'interaction entre les acides gras de la diète au niveau de l'estomac ou les acides biliaires (chargés négativement) avec le chitosane qui est chargé positivement. Le chitosane présente cependant l'inconvénient de sa source crustacée, potentiellement allergisante.

Les usages non alimentaires de compositions contenant le copolymère chitine-glucane sont connus, en particulier comme actif cicatrisant de la peau. Les compositions Mycoton et Mycoran issues d'*Aspergillus nigersont* décrites pour leurs propriétés par application sur la peau et sur les cicatrices. Cependant aucune application par voie orale n'est connue, en particulier dans le domaine alimentaire ou pharmaceutique.

La plupart des études menées sur les effets bénéfiques des fibres d'origine fongiques l'a été soit sur les champignons frais, soit sur des champignons en poudre, soit sur les beta-glucanes solubles en milieu aqueux extraits généralement de végétaux. Ces produits ne sont cependant pas les plus adaptés aux indications précitées. WO0135944 écrit l'utilisation de la chitosane (polysaccharide d'origine fongique) pour le traitement ia. de l'atherosclerose, l'hypercholestémie, dyslipidémie, hyperlipidémie et obésité. Le chitosane utilisé est un derivé déacetylé de la chitine.

### Buts de l'invention

Dans sa plus grande portée, la présente invention porte sur des objets tels que définis dans les revendications attachées. L'invention a pour but principal de fournir une famille de substances naturelles d'origine fongique qui permet de fournir un complément alimentaire ou une composition pharmaceutique, notamment pour améliorer la santé humaine et animale en complément d'une alimentation équilibrée et de bonnes pratiques d'hygiène, notamment dans la prévention et/ou la lutte contre des pathologies telles que le syndrome métabolique, l'obésité, le diabète et les maladies cardiovasculaires, ou les maladies associées.

La présente invention a également pour but de fournir une famille de substances qui présente une sécurité alimentaire irréprochable, tout en étant aisément disponible en grand volume à un coût compatible avec l'usage comme compléments alimentaires. La description a également pour but de fournir une famille de substances naturelles d'origine non animale, d'excellente pureté, bien caractérisées et avec une bonne traçabilité.

La description a également pour but de fournir un complément alimentaire, de type polysaccharide, stable et facile à formuler.

L'invention a pour but de proposer un actif pharmaceutique ou complément alimentaire permettant de ramener les paramètres associés aux pathologies du syndrome métabolique, de l'obésité, du diabète et/ou des maladies cardiovasculaires à leur niveau normal, comme, à titre d'exemple la teneur en triglycérides, l'équilibre entre le LDL- et le HDL-cholestérol, la surface de la crosse aortique recouverte de dépôts lipidiques, la capacité antioxydante du plasma, etc.

**Description de l'invention**

La présente invention décrit les effets de l'extrait, avantageusement purifié, de source fongique, et préférentiellement d'*Aspergillus niger,.* Les hydrolysats des extraits purifiés, c'est-à-dire les copolymères de chitine et beta-glucane de masse moléculaire inférieure, font également partie de l'invention. C'est l'association entre les deux polymères (chitine et beta-glucane) et l'architecture tridimensionnelle du copolymère issu de la source fongique qui en font un composé bénéfique pour la santé. La disponibilité et la qualité en particulier d'*Aspergillus niger,* qui est un coproduit de la production d'acide citrique à destination de l'industrie alimentaire et pharmaceutique, en font une matière première de choix pour les usages en santé humaine et animale de ses dérivés.

Des fibres de type polysaccharide et leurs oligomères d'origines diverses sont aussi connus pour leurs actions bénéfiques, comme les oligofructoses, la laminarine, le chitosane. Les présents inventeurs avaient déjà décrit un procédé de production de chitosane d'origine non animale, à partir de ressources fongiques, et plus particulièrement à partir d'un champignon de type *Aspergillus niger* dans la demande internationale WO 03/068824.

Or, les inventeurs ont découvert de manière surprenante que le composé chitine-glucane issu des sources de champignons mentionnés ci-dessus, et en particulier du champignon *Aspergillus niger,* et ses hydrolysats combinent de manière surprenante plusieurs des propriétés des fibres connues, et que le chitosane n'est pas le composé le plus indiqué pour pallier les dyslipidémies par exemple. En effet, le composé chitine-glucane et ses hydrolysats exercent à la fois des effets hypocholestérolémiant, hypoglycémique, ils augmentent la capacité antioxydante du plasma, favorisant ainsi la diminution de la plaque d'athérome, ils favorisent la satiété et stimulent le système immunitaire.

En ce qui concerne le chitosane, il existe l'a priori que son action amincissante s'exerce par sa capacité à lier les lipides au niveau gastrique, et que son action hypocholestérolémiante par sa capacité à lier les lipides au niveau intestinal, via des interactions électrostatiques entre les acides gras ou biliaire et les groupes ammonium du chitosane. Or, les substances chitine-glucane et ses hydrolysats de l'invention ne présentent d'une part essentiellement aucune charge électrostatique, et d'autre part ne sont pas capables de capter les acides gras *in vitro.* Néanmoins elles présentent une action réductrice significative de la teneur en triglycérides, de la teneur en cholestérol total et en LDL-cholestérol équivalente à celle du chitosane, tout en favorisant l'augmentation du HDL-cholestérol de manière équivalente à celle du chitosane.

A ces actions communes s'ajoutent des effets non décrits avec le chitosane, qui en font des substances avantageuses : le chitine-glucane et ses hydrolysats sont capables d'absorber généralement environ 10 fois leur masse en eau, ce qui en fait des substances avec de bonnes propriétés de fibres capables notamment d'améliorer le transit. Ils sont capables de stimuler une activité antioxydante du plasma de manière significative, et d'excercer une activité immunostimulante dûe à la synergie entre la partie chitine et la partie beta-glucane.

N'étant pas capables de capter les lipides au niveau gastrique, le chitine-glucane et ses hydrolysats n'entraînent pas les risques de déséquilibre en nutriments lipophiles (vitamines) comme cela est parfois suspecté pour le chitosane. Enfin, le chitine-glucane et ses hydrolysats sont obtenus et purifiés selon un procédé aisé, au départ de sources fongiques d'excellente qualité, qui sont des co-produits disponibles en grande quantité et renouvelable, et qui ne sont pas d'origine animale.

Les inventeurs entendent par « polysaccharides d'origine fongique » les extraits purifiés de parois cellulaires de champignons composés majoritairement de polysaccharides de chitine et de beta-glucane, sous forme de copolymères, et leurs hydrolysats. Les extraits purifiés comprennent une teneur de préférence en chitine-glucane supérieure à 70% en masse par rapport à la masse totale de l'extrait, de préférence supérieur à 80%, de préférence supérieure à 85% et encore de préférence supérieure à 95%.

Les inventeurs entendent par « chitine-glucane » un copolymère pur extrait des parois cellulaires de champignons qui est constitué de chaînons des unités N-acétyl-D-glucosamine et éventuellement d'une proportion minoritaire des unités D-glucosamine liées entre elles par des enchaînements de type (1,6) de conformation alpha (chaînon chitine), et de chaînons des unités D-glucose liées entre elles par des enchaînements de type (1,3), ou (1,3)(1,6), ou (1,3)(1,4), et préférentiellement (1,3), de conformation beta (chaînon beta-glucane, appelés ici « glucane »).

Il est généralement admis que les polysaccharides des parois cellulaires des champignons sont séparés en deux groupes selon leur solubilité en milieu alcalin, et que le squelette des parois cellulaires est insoluble. Il est également connu que la fraction insoluble est constituée de chitine et de beta-glucanes, en proportions variables selon les espèces, que les unités beta-glucanes sont liées par des enchaînement de structure variable, et que la liaison entre la chitine et les beta-glucanes est stable comme montré par exemple par Siestma & Wessels pour *Saccharomyces cerevisiae* (Zygomycete), *Neurospora crassa* (Ascomycete), *Aspergillus nidulans* (Ascomycete) et *Coprinus cinereus* (Basidiomycete) (Siestma JH & Wessels JG. (1981) Solubility of (1,3)-beta-D-(1,6)-beta-D-glucan in fungal walls : importance of presumed linkage between glucan and chitin. (1981) J. Gen. Microbiol. 125 :209). Il est connu que les chaînons chitine et beta-glucanes de la fraction insoluble d'*Aspergillus niger* sont liés entre eux de manière covalente, comme cité par exemple par Stagg CM et Feather MS (Biochim. Biophys. (1973) Acta 320:64). Des méthodes de détermination de la nature du lien covalent entre la chitine et les beta-glucanes ont été décrites par exemple par Fontaine et al. pour *Aspergillus fumigatus* (Fontaine T, Simenel C, Dubreucq G, Adam O, Delepierre M, Lemoine J, Vorgias CE, Diaquin M & Latgé JP. (2000) Molecular organization of the alkali-insoluble fraction of Aspergillus fumigatus cell wall, J. Bio. Chem. 275:27594), et par Kollar et al. pour la levure *Saccharomyces cerevisiae* (Kollar R, Petrakovas E, Ashwell G, Robbins P & Cabib E. (1995) Architecture of the yeast cell wall, the linkage between chitin and beta(1,3)glucan, J. Biol. Chem. 270:1170).

Avantageusement le copolymère chitine-glucane contient moins de 10 % de composés solubles dans l'eau. De préférence, le copolymère chitine-glucanes a une pureté supérieure à 80 %, et de préférence supérieure à 85 %. Avantageusement, le copolymère chitine-glucanes contient moins de 40 mg/kg of métaux lourds, tel que déterminé par la méthode décrite dans la Pharmacopée Européenne (monographie 2.4.8F), et avantageusement moins de 20 mg/kg de métaux lourds. Avantageusement le copolymère chitine-glucane contient moins de 2 ppm d'arsenic, comme déterminé par AES-ICP. Avantageusement, le chitine-glucane a une qualité microbiologique adaptée à l'utilisation alimentaire, et de préférence contient moins de 10,000 cfu/g, et de préférence moins de 1,000 cfu/g, pour le compte total de micro-organismes, y compris les levures et moisissures.

Le rapport entre la chitine et le beta-glucane est compris entre 95:5 et 5:95, de préférence entre 70:30 et 20:80, et de préférence encore entre 70:30 et 25:75, et de préférence encore entre 60:40 et 25:75 (m/m). Un autre rapport entre la chitine et le beta-glucane préféré est compris entre 50:50 et 10:90, de préférence entre 45:55 et 20:80. Ce type de copolymère peut être obtenu par hydrolyse du copolymer chitine-glucan décrit précédemment. La partie chitine du copolymère chitine-glucane est composée de préférence d'au moins 85% d'unités N-acétyl-D-glucosamine et d'au plus 15% d'unités D-glucosamine, préférentiellement d'au moins 90% d'unités N-acétyl-D-glucosamine et d'au plus 10% d'unités D-glucosamine. Le copolymère se présente généralement sous la forme d'une poudre blanche à légèrement brune. Il est essentiellement insoluble dans les solvants aqueux et organiques quelle que soit la température et le pH. Il est capable de gonfler dans les milieux aqueux. Il est hygroscopique, pouvant généralement absorber environ 7 fois sa masse en eau, ce qui correspond à la capacité de gonflement des fibres végétales insolubles comme l'hémicellulose ou la pectine.

Avantageusement, les copolymères chitine-glucane utilisés dans la présente invention sont obtenus par le procédé décrit dans la demande internationale WO 03/068824 et la demande de brevet Français FR 0507066 déposée au nom de KITOZYME S.A. respectivement le 12 février 2003 et le 4 juillet 2005. Ce procédé est décrit en particulier dans la demande FR 0507066 pages 18, ligne 14, et suivantes de la demande telle que déposée. On utilise de préférence *Aspergillus niger* comme source fongique dans ce procédé.

Un procédé préféré comprend les étapes suivantes :

1) éventuellement suspendre la biomasse dans une solution acide et retirer les produits solubles dans le milieu acide,
2) suspendre à la fraction insoluble en milieu acide dans d'une solution alkaline et retirer les composés solubles en milieu alcalin,
3) purifier le produit insoluble en milieu alcalin par un traitement supplémentaire avec de l'eau,
4) sécher le produit insoluble dans l'eau,
5) éventuellement purifier le produit séché par un traitement avec un solvant organique,
6) sécher le produit insoluble en milieu organique.

La première étape de suspension de la biomasse dans une solution acide est optionnelle. Elle dépend de la composition de la biomasse initiale et de la pureté requise pour le produit copolymère chitine-glucane final. La solution acide est de préférence une solution aqueuse d'acide chlorhydrique ou d'acide sulfurique, et de préférence d'acide chlorhydrique. Selon un mode de réalisation particulier, la solution acide a une concentration comprise entre 0,1 et 5 N, et de préférence d'environ 0,5 N.

La première étape d'extraction permet avantageusement d'éliminer les composés solubles en milieu acide, incluant les composés inorganiques.

La seconde étape met avantageusement en oeuvre une solution aqueuse comme une solution aqueuse d'hydroxyde de sodium, d'hydroxyde de potassium, ou d'hydroxyde d'ammonium, et de préférence l'hydroxyde de potassium ou de sodium. Selon un mode de réalisation préféré, la solution basique a une concentration comprise entre 0,1 et 5 N, et de préférence d'environ 1 N.

La troisième étape de purification du produit insoluble en milieu alcalin est avantageusement réalisée par mise en contact de cette fraction avec de l'eau et séparation des produits insolubles dans l'eau par filtration.

Chaque étape peut être réalisée à une température de préférence comprise entre 5 et 120° C et de préférence à une température inférieure à 60° C. La biomasse est de préférence comprise entre 1 et 15 % (en masse sèche, m/v), et encore de préférence entre 3 et 12 %. Les premières et seconde étapes durent de préférence entre 4 et 48 heures, et de préférence moins de 30 heures. Chaque étape peut être répétée plusieurs fois.

Chaque étape peut être répétée plusieurs fois.

Les inventeurs entendent par « hydrolysats de chitine-glucane » les copolymères résultants de l'hydrolyse contrôlée du copolymère chitine-glucane extrait notamment de parois cellulaires de champignons, qui sont également constitués de chaînons chitine et de chaînons beta-glucanes, liés de manière covalente, avec le rapport entre la chitine et les beta-glucane de préférence compris entre 50:50 et 10:90 (m/m), de préférence entre 60:40 et 15:85 (m/m). Les hydrolysats présentent une masse moléculaire inférieure à celle du copolymère chitine-glucane, résultant d'une hydrolyse partielle. La partie chitine des hydrolysats de chitine-glucane est composée de préférence d'au moins 85% d'unités N-acétyl-D-glucosamine et d'au plus 15% d'unités D-glucosamine, préférentiellement au moins 90% d'unités N-acétyl-D-glucosamine et de au plus 10% d'unités D-glucosamine.

### Description détaillée de l'invention

Les inventeurs ont découvert de manière surprenante qu'un copolymère chitine-glucane et ses hydrolysats appartenant à une famille de polysaccharides d'une structure proche de celle du chitosane, présentent des propriétés hypocholestérolémiante équivalentes à celle du chitosane, bien qu'il ne soit essentiellement pas chargé. Ce composé est obtenu aisément au départ de sources fongiques, comme par exemple de mycelium d'*Aspergillus niger,* dont il constitue la partie insoluble de l'exosquelette des parois cellulaires. Le copolymère chitine-glucane et ses hydrolysats présentent également d'autres effets bénéfiques pour la santé, décrits dans la présente invention.

L'invention concerne en particulier l'utilisation de polysaccharides extraits de sources fongiques et leurs dérivés hydrolysés comme actifs pharmaceutiques ou compléments alimentaires pour améliorer la santé humaine et animale. L'administration orale régulière des poysaccharides de l'invention permet en particulier de prévenir, traiter ou lutter contre notamment la dyslipidémie, l'hypercholestérolémie, une athérosclérose, et de stimuler les activités antioxydantes de l'organisme, stimuler le système immunitaire, exercer une action hypoglycémique favorable dans les cas de diabète, et favoriser la satiété pour minimiser le syndrome métabolique. Les polysaccharides utilisés dans l'invention sont obtenus à partir de sources fongiques selon un procédé avantageux, permettant une excellente pureté, une bonne reproductibilité, avec une capacité de production importante. Les polysaccharides utilisés dans l'invention sont constitués essentiellement des unités D-glucosamine, et/ou N-acétyl-D-glucosamine et D-glucose, en particulier des polymères chitine (N-acétyl-D-glucosamine) et beta-glucane (D-glucose). La description comprend également toutes modifications du polymère ou copolymère, par exemple par greffage des fonctions chimiques sur le polymère, pour en améliorer les propriétés.

Ainsi, la description concerne selon un premier aspect l'utilisation d'au moins un polysaccharide d'origine fongique comprenant majoritairement un copolymère chitine-glucane, pour la fabrication d'une composition administrée par voie orale.

La description concerne également l'utilisation d'au moins un polysaccharide d'origine fongique insoluble en milieu aqueux et organique, ledit polysaccharide comprenant majoritairement un copolymère chitine-glucane, pour la fabrication d'une composition administrée par voie orale.

La description concerne également l'utilisation d'au moins un polysaccharide d'origine fongique comprenant un polymère comprenant des enchaînements de beta-glucanes, lesdits enchaînements de beta-glucanes consistant essentiellement d'enchaînements de beta-glucanes en position 1,3, pour la fabrication d'une composition administrée par voie orale.

La description concerne également l'utilisation d'au moins un extrait d'origine fongique contenant essentiellement un polysaccharide tel que défini précédemment, pour la fabrication d'une composition administrée par voie orale. Avantageusement le polysaccharide comprend plus de 70 % de polysaccharides chitine-glucane en masse par rapport à la masse totale de l'extrait d'origine fongique, de préférence supérieure à 85%.

L'extrait fongique peut être obtenu à partir de paroi cellulaire de mycélium fongiques de différents groupes, incluant Zygomycetes, Basidiomycetes, Ascomycetes (dont *Aspergillus niger* fait partie) et Deuteromycetes et/ou un mélange de ceux-ci. Ladite source de champignons doit être choisie de manière à permettre l'extraction d'un polysaccharide tel que défini ci-dessus et ci-après. Il existe des sources de champignons qui comprennent des glucanes, mais ces unités sont solubles dans l'eau notamment, ou ne comprennent pas ou peu de chaînes de structure chitine, et ne permettent donc pas d'obtenir le polysaccharide de la présente invention. La source fongique peut être aussi bien naturelle que génétiquement modifiée (OGM).

La description couvre l'ensemble des champignons qui permettent d'obtenir le polymère chitine-glucane défini dans la présente demande.

Avantageusement, on utilise un hydrolysat du polysaccharide défini précédemment.

Avantageusement, l'hydrolysat de chitine-glucane a un rapport entre la chitine et les beta-glucanes compris entre 60:40 et 15:85 (m/m).

Avantageusement, au moins 85% de la partie chitine du copolymère chitine-glucane sont des unités N-acétyl-D-glucosamines, et au plus 15% de cette partie sont des unités D-glucosamines.

Ainsi, l'invention permet de fournir une composition administrée par voie orale à un être humain ou un animal, de préférence un mammifère, pour obtenir un effet choisi parmi le groupe consistant en un effet antioxydant, hypocholestérolémiant, hypolipidémiant, hypoglycémique, notamment dans le cas de diabète, de satiété, d'amélioration du transit alimentaire, et un effet de prévention et/ou de traitement, et/ou de lutte contre une pathologie choisie parmi le groupe consistant en la dyslipidémie, l'athérosclérose, l'obésité, une maladie cardio-vasculaire, le syndrome métabolique, le diabète, et l'hyperuricémie. La description concerne également une composition pharmaceutique ou de complément alimentaire comprenant à titre de principe actif au moins un polysaccharide ou un extrait d'origine fongique, tel que défini précédemment.

La description concerne également une méthode de traitement, de prévention, ou de lutte contre une pathologie, notamment celle mentionnée ci-dessus, comprenant l'administration par voie orale d'une quantité efficace un sujet en ayant besoin d'une composition comprenant au moins un polysaccharide tel que défini dans la description ci-dessus et ci-après.

La présente invention concerne également une méthode pour diminuer la masse ou prévenir ou lutter contre la prise de masse d'un être humain ou un animal, et de préférence un mammifère. Cette méthode concerne un soin esthétique.

La présente invention concerne également une méthode pour favoriser le transit alimentaire.

Ainsi, la présente invention concerne l'utilisation d'un produit de la présente invention pour la fabrication d'une composition destinée notamment à être utilisée dans l'une des méthodes décrites ci-dessus ou pour exercer l'un des effets décrits ci-dessus et ci-après.

L'homme du métier détermine aisément par les méthodes classiques les quantités efficaces des produits de l'invention à utiliser. On utilise avantageusement une quantité efficace comprise entre 0.001 et 100% en poids de des produits selon la présente invention par rapport au poids total de la composition à administrer. Si les produits sont administrés sous forme de gélules, granules, ou comprimés, ils peuvent être utilisés purs ou à toute autre concentration, accompagnés d'autres composants actifs ou d'excipients. Si ils sont incorporés dans des aliments, la concentration en produit est inférieure à 15%, et de préférence inférieure à 10%.

Les produits utilisés dans l'invention sont généralement formulés sous forme de granules, de comprimés, de gélules, ou encore incorporés à des aliments ou à des boissons.

Sur les figures :

La figure 1 représente les conditions d'enregistrement du spectre de résonance magnétique nucléaire du carbone 13 (13C-RMN) en phase solide de chitine-glucan F1 et chitine-glucan hydrolysé F4.
La figure 2 représente le spectre de 13C-RMN de chitine-glucan F1 (lot 28).

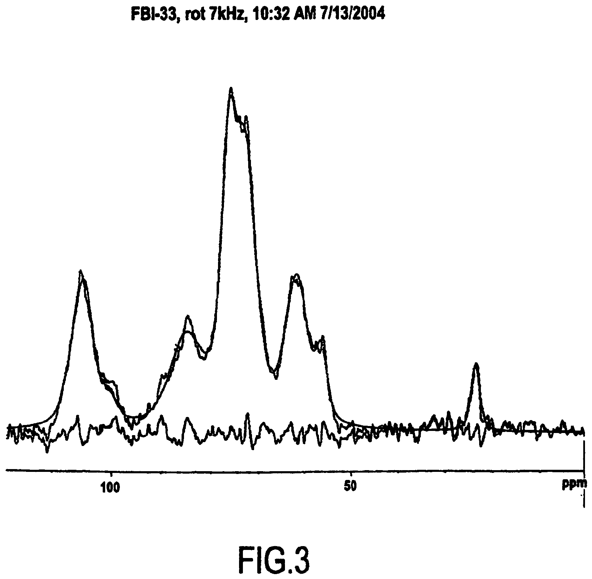

La figure 3 représente le spectre de 13C-RMN du chitine-glucan hydrolysé F4 (lot 2).

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en poids, sauf indication contraire, et la température est exprimée en degré Celsius sauf indication contraire, et la pression est la pression atmosphérique, sauf indication contraire.

## Exemples

Le procédé d'obtention des copolymères chitine-glucane obtenus ci-dessous est décrit dans les demandes de brevets WO 03/068824 et FR 05.07066.

**EXEMPLE 1-** Exemples de copolymères chitine-glucane extraits et purifiées du mycelium d'*Aspergillus niger* (extrait F1)

Pour préparer le copolymère chitine-glucane F1, une masse de 50 kg (poids sec) de mycelium d'*Aspergillus niger* humide est mise en suspension dans une solution d'acide chlorhydrique à une concentration de 1%, puis filtrée. La matière solide est ensuite mise en suspension dans une solution d'hydroxyde de sodium à 0.25%, puis filtrée. La matière solide est lavée 4 fois à l'eau, puis séchée. Elle est ensuite mise en suspension dans l'éthanol, puis filtrée et séchée. On obtient environ 20 kg de chitine-glucane (F1).

Les caractéristiques moléculaires et la composition de six lots de chitine-glucane F1 sont données dans le Tableau 1.

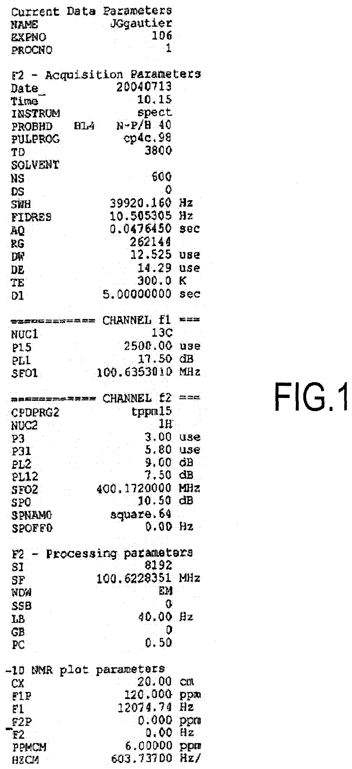

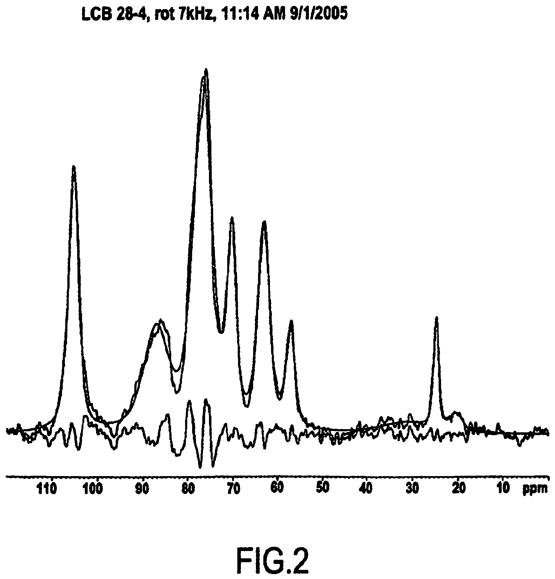

Le rapport massique chitine/glucane est calculé à partir du spectre de résonance magnétique nucléaire (RMN) du carbone 13 en phase solide enregistré dans les conditions indiquées à la Figure 1 selon la méthode décrite brièvement ci-dessous. Le spectre du composé chitine-glucane F1 (lot 28) est présenté à la Figure 2. La proportion en beta-glucan est déterminée à partir de l'aire des quatres bandes de résonance suivantes : 104 ppm (carbone 1 de la chitine et du beta-glucane), 23 ppm (carbone CH$_3$ de la chitine), 55 ppm (carbone 2 de la chitine) et 61 ppm (carbone 6 de la chitine et du beta-glucane), en prenant comme référence la chitine pure. Par exemple, on peut effectuer le calcul selon la Formule 1, où l'est l'aire des signaux des carbones, et où [ ]$_{CG}$ indique la valeur du rapport pour le chitine-glucan analysé et [ ]$_C$ pour la chitine de référence. C1 est le carbone 1 de la chitine et du beta-glucane et C2 est le carbone 2 de la chitine.

$$\text{Glucan (mol\%)} = \frac{[\frac{I'(C1)}{I'(C2)}]_{CG} - [\frac{I'(C1)}{I'(C2)}]_C}{[\frac{I'(C1)}{I'(C2)}]_{CG}} \times 100 \qquad (1)$$

Le rapport massique chitine/glucan des six lots de chitine-glucan est en moyenne de 39:61 $\pm$ 2 (m/m).

La proportion d'unités D-glucosamine (NGlc), exprimée en % molaire de la partie chitine, peut être estimée à partir du spectre RMN, comme décrit par Heux et al. (Heux L, Brugnerotto J, Desbrières J, Versali MF & Rinaudo M. (2000) Solid state NMR for determination of the degree of acetylation of chitin and chitosan. Biomacromolecules 1:746). La proportion d'unités D-glucosamine est déterminée par titrage potentiométrique par l'hydroxyde de sodium, en suspension dans un excès d'acide chlorhydrique.

La qualité microbiologique du chitine-glucane et les résultats de recherche d'agents pathogènes sont donnés dans le Tableau 2.

**Tableau 1-** Caractéristiques moléculaires et composition de différents lots du copolymère chitine-glucane (F1)

| Lot | Rapport chitine-glucane | NGlc (titrage) | Cendres | Protéines | Lipides | Métaux lourds |
|---|---|---|---|---|---|---|
| | (m/m) | mol% | (%) | (%) | (%) | (ppm) |
| F1 lot 26 | 36:64 $\pm$ 5* | 0 | 0,4 | 4,63 | 0 | < LQ** |
| F1 lot 27 | 42:58 $\pm$ 7 | 0 | 1,3 | 3,54 | 0 | < LQ |
| F1 lot 28 | 39:61 $\pm$ 7 | 0 | 1,5 | 2,51 | 2,09 | < LQ |
| F1 lot 29 | 40:60 $\pm$ 6 | 0 | 1,7 | 3,05 | 0,06 | < LQ |
| F1 lot 30 | 37:63 $\pm$ 1 | 0 | 1,9 | 1,54 | 1,24 | 8,7 |
| F1 lot 31 | 40:60 $\pm$ 4 | 0 | 2,5 | 4,26 | 1,27 | < LQ |
| *écart-type sur le résultat de 4 calculs du rapport chitine-glucan ; **LQ : 5 limite de sensibilité de la méthode d'analyse par Ion Coupled Plasma (5.3 ppm) | | | | | | |

**Tableau 2-** Qualité microbiologique du chitine-glucane F1 (lot 26)

| | Nombre de germes/g |
|---|---|
| **Germes totaux aérobies mésophiles** | 20 cfu/g |
| **Spores aérobies** | < 10 cfu/g |
| **Levures et moisissures** | 20 cfu/g |
| **Agents pathogènes** | |
| *Enterobacteriaceae* | < 10 cfu/g |
| *Escherichia coli* | < 10 cfu/g |
| *Staphylococcus coagulase+* | < 10 cfu/g |

| | Nombre de germes/g |
|---|---|
| *Pseudomonas spp* | 10 cfu/g |
| *Salmonella spp* | Absence |

On comprend ainsi des tableaux précédents que le copolymère selon la présente invention à un degré de pureté élevé.

**EXEMPLE 2-** Exemples d'hydrolysats du copolymère chitine-glucane (F4)

Dans cet exemple, les hydrolysats de chitine-glucane F4 sont obtenus par hydrolyse basique du copolymère chitine-glucane F1 à 100°C ou au delà, pendant au moins 2 heures, dans une solution d'hydroxyde de sodium de concentration supérieure à 30%. La matière est lavée à l'eau plusieurs fois, mise en suspension dans l'éthanol, puis filtrée et séchée. On obtient environ 5 kg d'hydrolysat au départ de 20 kg de chitine-glucane F1.
Les caractéristiques moléculaires et la composition de quelques lots d'hydrolysats de chitine-glucane F4 sont données dans le Tableau 3. Le rapport chitine/glucane est calculé à partir du spectre de résonance magnétique nucléaire du carbone 13 en phase solide. Le rapport est variable selon la méthode d'hydrolyse employée. Les conditions d'analyses sont identiques à celles utilisées pour F1.

**Tableau 3-** Caractéristiques moléculaires et composition (cendres, protéines) de différents lots d'hydrolysats de chitine-glucane (F4)

| | Rapport chitine-glucane | NGlc (titrage) | Cendres | Protéines |
|---|---|---|---|---|
| | (m/m) | mol % | % | % |
| F4 lot 1 | 26:74 $\pm$ 3 | 0.2 | ND | 0.1 |
| F4 lot 2 | 25:75 $\pm$ 2 | 0 | 1.2 | 8.9 |
| F4 lot 3 | 61:39 $\pm$ 3 | 0 | 5.3 | 3.9 |
| *écart-type sur le résultat de 4 calculs du rapport chitine-glucan* | | | | |

Le copolymère selon la présente invention a bien un degré de pureté élevé.

**EXEMPLE 3- Mise en évidence de l'effet anti-athérosclérose, antioxydant, hypocholestérolémiant, hypolipidémiant, après administration orale de chitine-glucane hydrolysé**

Le modèle animal utilisé est le hamster sous régime athérogénique. Les hamsters sont nourris avec une diète enrichie en cholestérol, et déficiente en antioxidants (vitamine C, vitamine E et sélénium), ce qui entraîne une dyslipidémie et des lésions artérielle similaires aux lésions rencontrées au niveau des plaques d'athérome chez l'homme (Nistor A, Bulla A, Filip DA & Radu A (1987) The hyperlipidemic hamster as a model of experimental atherosclerosis. Atherosclerosis 68:159). Le hamster représente un modèle animal d'athérosclérose particulièrement intéressant à cause de son métabolisme lipidique, sa manipulation aisée et le faible temps nécessaire à l'induction des lésions (de 8 à 12 semaines). Il a été choisi pour étudier les effets d'un hydrolysat de chitine-glucane car il répond bien aux interventions visant à diminuer le cholestérol et l'athérome (Kowala, MC, Nunnari, JJ, Durham, SK & Nicolosi, RJ (1991) Doxazosin and cholestyramine similarly decrease fatty streak formation in the aortic arch of hyperlipidemic hamsters. Atherosclerosis 91:3549).
Dans cet exemple, deux composés dont les caractéristiques sont résumées dans le Tableau 4 ont été administrés dans la diète quotidienne des hamsters :

- 42.85 mg/kg/jour de chitine-glucane hydrolysé F4 (soit 3 g/jour pour un homme de 70 kg)
- 42.85 mg/kg/jour de chitosane d'origine fongique F7 (soit 3 g/jour pour un homme de 70 kg)

Le groupe témoin reçoit de l'eau par gavage journalier afin d'obtenir les mêmes conditions expérimentales que les autres groupes et éviter d'éventuelles différences dues au stress du gavage.
Dès leur réception, 28 hamsters dorés syriens d'environ 80 grammes, sont placés dans des cages en plastique, divisés en trois lots de huit animaux dans une pièce à 23°C, avec une hygrométrie de 70% et sous photopériode de 12 heures (12N/12D). La consommation alimentaire et leurs poids sont mesurés tous les jours. Les groupes reçoivent le régime dont la composition est détaillée dans le Tableau 5.

**Tableau 4-** Caractéristiques des composés F4 et F7

|  | **F4** Chitine-glucane hydrolysé | **F7** Chitosane |
|---|---|---|
| Masse moléculaire | N/A | 10000 |
| Chitine (% du copolymère chitine-glucane) | 25 | NA |
| Beta-glucane (% du copolymère chitine-glucane) | 75 | 2 |
| Glucosamine (mol% de la partie chitine) | 0 | 90 |
| Cendres (%) | 1,2 | 1,3 |
| Protéines (%) | 8,9 | <1 |

**Tableau 5** - Composition du régime des hamsters

| Ingrédients | (g/kg) |
|---|---|
| Caséine | 200 |
| DL-méthionine | 3 |
| Amidon de blé | 393 |
| Saccharose | 154 |
| Cellulose | 50 |
| Huile de maïs | 25 |
| Huile de colza | 25 |
| Mélange minéral [1] | 35 |
| Mélange vitaminique [2] | 10 |
| Saindoux | 150 |
| Cholestérol | 5 |

[1]*Le mélange minéral contient (mg/kg régime): $CaHPO_4$, 17200; $KCl$, 4000; $NaCl$, 4000; $MgO$, 420; $MgSO_4$, 2000; $Fe_2O_3$, 120; $FeSO_4.7H_2O$, 200; éléments trace, 400 ($MnSO_4.H_2O$, 98; $CuSO_4.5H_2O$, 20; $ZnSO_4.7H_2O$, 80; $CoSO_4.7H_2O$, 0,16; $KI$, 0,32; amidon, qsp. 40 g (par kg régime). Ce mélange est dépourvu de $Na_2SeO_3$.*
[2]*Mélange vitaminique contenant (mg/kg régime): rétinol, 12; cholécalciférol, 0.125; thiamine, 40; riboflavine, 30; acide pantothenique, 140; pyridoxine, 20; inositol, 300; cyanocobalamine, 0.1; ménadione, 80; acide nicotinique, 200; choline, 2720; acide folique, 10; acide p-aminobenzoique, 100; biotine, 0, 6; amidon, qsp 20 g (par kg régime). Ce mélange est dépourvu d'alpha-tocophérol et d'acide ascorbique.*

**Prélèvement des tissus.** Après une période expérimentale de 12 semaines, les hamsters sont placés 16 heures à jeun puis sont anesthésiés par voie intrapéritonéale.

**Prélèvement des plasmas.** Le sang est d'abord prélevé par ponction intracardiaque puis est centrifugé à 3500xg pendant 10 minutes. Le plasma est récupéré, aliquoté puis conservé à -80°C. Sur ces plasmas seront déterminés par la suite le cholestérol total, le taux de cholestérol lié aux HDL, le taux de cholestérol lié aux LDL, les triglycérides, l'acide urique et l'urée.

**Prélevement des organes.** On prélève le foie après perfusion par une solution de tampon phosphate salin (1 mM, pH 7,2) contenant du chlorure de calcium 1mM et 0,27% de glucose. Ceci a pour but d'éliminer le sang résiduel qui pourrait comporter une activité SOD et GSHPx. Celui-ci est ensuite séché, pesé puis conservé à -80°C. L'activité de la glutathion peroxydase et de la superoxyde dismutase seront déterminées sur cet organe.

L'aorte est prélevée ensuite après fixation de l'appareil vasculaire par un tampon cacodylate de sodium 0,1 M, pH 7.4, contenant du chlorure de calcium 2,5 mM, 2,5% de paraformaldéhyde et 1,5% de glutaraldéhyde. La crosse aortique est ensuite prélevée sous une loupe binoculaire, ouverte longitudinalement, épinglée sur un morceau de liège puis immergée dans la solution de fixation et stockée à 4°C. Les lésions athéromateuses provoquées par le régime pourront être observées après coloration des lipides de la paroi aortique.

**Traitement statistique des résultats**

Les valeurs qui seront présentés dans la partie suivantes correspondent au moyenne $\pm$ SEM (erreur standard à la moyenne) obtenus sur des groupes de 8 animaux. La significativité entre les moyennes a été établie par une analyse à une variable ANOVA utilisant un test de Fischer à l'aide du logiciel StatView 4.5 (ABACCUS Concept, Inc). Quand les valeurs sont affectées d'une lettre identique en exposant, cela signifie que ces valeurs ne sont pas significativement différentes pour P <0,05.

**Résultats**

**a- Détermination de la surface lipidique.**

La surface des dépôts lipidiques est déterminée à l'aide d'un microscope optique muni d'un appareil photo, après coloration histologique selon la méthode décrite par Nunnari et al. (Nunnari JJ, Zand T, Joris I & Majno G. (1989) Quantification of oil Red O staining of the aorta in hypercholesterolemic rats. Exp. Mol. Pathol. 51:1).

**Tableau 6-** Pourcentage de surface aortique recouverte de dépôts lipidiques aortiques

|  | Témoin | F4 | F7 |
|---|---|---|---|
| Pourcentage dépôts lipidiques | 14,79 $\pm$ 4,08[a] | 0,35 $\pm$ 0,20[b] | 0,57 $\pm$0,37[b] |

On constate que le produit selon la présente invention est plus efficace que le chitosane.

**b- Dosages plasmatiques**

Plusieurs dosages sont effectués sur les plasmas :

- Bilan biologique (cholestérol total, triglycérides, HDL, LDL, acide urique, urée)
- Dosage de la capacité antioxydante (TAS)

**Détermination du cholestérol plasmatique total.** L'analyse a été réalisée avec le Kit No. 1489232 Roche/Hitachi, Roche Diagnostics. Le dosage repose sur la technique décrite par Allain et al. (Allain CC, Poon LS, Chan CS, Richmond W & Fu PC. (1974) Enzymatic determination of total serum cholesterol, Clin. Chem. 20:470).

**Détermination des triglycérides plasmatiques.** L'analyse a été réalisée avec le Kit No. 1488872 Roche/Hitachi, Roche Diagnostics, selon la méthode mise au point par Wahlefeld & Bergmeyer (Wahlefeld AW & Bergmeyer HU (1974) in : Methods of enzymatic analysis Second Edition, New York Academic Press p.1831).

**Détermination des HDL plasmatiques.** L'analyse a été réalisée avec le Kit No. 1930672 Roche/Hitachi, Roche Diagnostics. La séparation des HDL se fait selon la technique décrite par Marz et Gross (Mârz W & Gross W. (1986) Evaluation of a phosphotungstic acid/MgCl2 precipitation and quantitative lipoprotein electrophoresis assay. Clin. Chim. Acta. 158:33).

**Détermination des LDL plasmatiques.** La teneur plasmatique en LDL-cholestérol est calculée par différence entre la teneur en cholestérol total et la teneur en HDL-cholestérol.

**Tableau 7-** Bilan des paramètres biochimiques

|  | Témoin | F4 | F7 |
|---|---|---|---|
| Cholestérol total (g/L) | 3,10 $\pm$ 0,30a | 2,53 $\pm$ 0,21b | 2,37 $\pm$ 0,03b |
| Triglycérides (g/L) | 1,96 $\pm$ 0,55a | 1,04 $\pm$0,27bd | 0,60 $\pm$ 0,21bc |
| HDL (g/L) | 0,57 $\pm$ 0,16a | 2,01 $\pm$0,11bd | 1,84 $\pm$0,09bc |
| LDL (g/L) | 2,13 $\pm$ 0,2a | 0,24 $\pm$ 0,05b | 0,26 $\pm$ 0,09b |

On constate que les produits de la présente invention ont un effet équivalent au chitosane.

**Détermination de l'urée.** L'analyse a été réalisée avec le Kit No. 1489364 Roche/Hitachi, Roche Diagnostics. Ce dosage de l'urée repose sur une méthode UV cinétique décrite par Talke et Schubert (Talke H & Schubert GE. (1965) Enzymatische Harnstoffbest timmung im blut und serum im optischen test nach Warburg. Klin. Wochenschr. 43:174).

**Détermination de l'acide urique.** L'analyse a été réalisée avec le Kit COBAS INTEGRA 400/700/800 Uric Acid ver.2 (UA2) Roche/Hitachi, Roche Diagnostics. Il s'agit d'un test colorimétrique enzymatique selon la méthode modifiée de Town et al. (Town MH et al. (1985) J. Clin. Chem. Clin. Biochem. 23:591).

**Tableau 8-** Activités enzymatiques du plasma

|  | Témoin | F4 | F7 |
|---|---|---|---|
| Acide urique (µmol/L) | 255,00 ± 37,85[a] | 100,75 ± 62,02[bd] | 82,25 ± 21,70[bc] |
| Urée (mmol/L) | 14,87 ± 5,58[a] | 4,00 ± 0,37[b] | 5,25 ± 0,25[b] |

On constate que l'activité enzymatique du plasma des produits de la présente invention est meilleure ou équivalente à celle du chitosane.

**Dosage de la capacité antioxydante du plasma (TAS).** Ce paramètre est déterminé en utilisant le Kit Randox No.NX2332 (Laboratoire Randox), dont la méthode repose sur la technique de Miller et al. (Miller NJ, Rice-Evans C, Davies MJ, Gopinathan V & Milner A. (1993) A novel method for measuring antioxydant capacity and its application to monitoring the antioxydant status in premature neonates, Clinical Science. 84:407).

**Tableau 9-** Activité antioxydante du plasma

|  | Témoin | F4 | F7 |
|---|---|---|---|
| Capacité antioxydante (mmol/L) | 1,03 ± 0,08[abd] | 1,37 ± 0,07[bce] | 0,89 ± 0,04[de] |

On constate que les produits de la présente invention ont une activité antioxydante du plasma tout à fait surprenant et non comparable à celle du chitosane qui inhibe l'activité antioxydante du plasma.

**c- Dosage des activités enzymatiques hépatiques**

**Préparation des fractions cytosoliques de foie.** L'activité enzymatique hépatique est mesurée sur des fractions cytosoliques; en effet, les enzymes se situant dans le cytosol, il est nécessaire de procéder à une ultracentrifugation en deux étapes : une première centrifugation des homogénats de foie à 5% dans le NaCl 0,15M, à 8000 x g pendant 20 minutes à 4°C ce qui permet de récupérer un surnageant. Ce dernier subit alors une ultracentrifugation à 105 000 x g pendant une heure à 4°C. Le cytosol est conservé à -80°C en vue des dosages. Le dosage des protéines est effectué par la méthode à l'acide bincinchoninique, selon Smith et al. (Smith PK et al. (1985) Measurement of protein using bicinchoninic acid. Anal. Biochem. 150:76).

**Activité de la glutathion peroxydase (Se-GSHPx).** La mesure d'activité de cette enzyme est basée sur la capacité de l'échantillon à catalyser l'oxydation du glutathion par l'eau oxygénée, selon la méthode de Wendel (Wendel A. (1981) Glutathione peroxydase. Methods in enzymology, 77:327)

**Tableau 10-** Activité spécifique de la Se-GSHPx hépatique

|  | Témoin | F4 | F7 |
|---|---|---|---|
| Se-GSHPx (U/mg protéines) | 109,12 ± 88,94[a] | 134,04 ± 1,49[a] | 142,85 ± 54,60[a] |

On constate que les produits de la présente invention ont une activité enzymatique hépatique équivalente à celle du chitosane.

**Activité de la superoxyde dismutase (SOD).** L'activité de la SOD est déterminée avec le kit SOD 525 (Tebu-Bio) selon la méthode de Paoletti et Mocali (Paoletti F & Mocali A. (1990) Determination of superoxyde dismutase activity by purely chemical system based on NADCPJH oxidation, Methods Enzymology. 186:209).

**Tableau 11 :** Activité spécifique de la SOD hépatique

|  | Témoin | F4 | F7 |
|---|---|---|---|
| SOD (U/mg protéines) | 0,58 ± 0,56[a] | 0,03 ± 0,02[bd] | 0,04 ± 0,01[cd] |

On constate que les produits de l'invention ont une activité sur la superoxyde dismutase comparable à celle du chitosane.

Pour résumer les résultats, les Tableaux 12 et 13 rassemblent les variations des différents paramètres exprimées en % par rapport au groupe témoin

**Tableau 12-** Variations de la surface des dépôts lipidiques aortiques et des activités enzymatiques hépatiques exprimées en % par rapport au groupe témoin.

|  | Se-GSHPx (U/mg protéines) | SOD (U/mg protéines) | Pourcentage dépôts lipidiques |
|---|---|---|---|
| F4 | +22,8% | -94,8% | -97,6% |
| F7 | +30,9% | -93,1% | -96,1% |

**Tableau 13-** Variations des différents paramètres biochimiques exprimées en %, par rapport au groupe témoin.

|  | Cholestérol total (g/L) | Triglycérides (g/L) | HDL (g/L) | LDL (g/L) | Capacité antioxydante (mmol/L) | Acide urique (μmol/L) | Urée (mmol/L ) |
|---|---|---|---|---|---|---|---|
| F4 | -18,4% | -46,9% | +252,6% | -88,7% | +33,1% | -60,5% | -73,1% |
| F7 | -23,5% | -69,4% | +222,8% | -87,8% | -13,6% | -67,7% | -64,7% |

Le cholestérol total, les triglycérides, le HDL-cholestérol et le LDL-cholestérol sont des paramètres biochimiques qui permettent de constater les effets anti-cholestérolémiant du du chitine-glucane hydrolysé et du chitosane. En effet, la diminution du cholestérol total et du LDL-cholestérol (diminution significative d'un facteur 10) par rapport au groupe témoin permettent de confirmer l'efficacité du chitine-glucane hydrolysé et du chitosane dans la prévention du développement de la plaque d'athérome et par voie de conséquence sur les maladies cardiovasculaires.

L'augmentation du HDL-cholestérol est spectaculaire. Elle traduit une favorisation de l'efflux du cholestérol des organes périphériques vers le foie, diminuant ainsi le risque de dépôts et d'oxydation des particules riches en cholestérol dans les organes et les artères. Les traitements à base de F4 et F7 permettent d'inverser les niveaux d'HDL/LDL par rapport au témoin.

Le pourcentage de la surface de la crosse aortique recouverte par les dépôts lipidiques et les cellules spumeuses est un indicateur direct du développement des lésions athéromateuses. Par rapport aux témoins, les deux types de traitement réduisent de façon très significative ce pourcentage, avec un niveau d'efficacité quasi similaire autour de 97%. Or, des composés réputés très efficaces pour la réduction des dépôts lipidiques comme les polyphénols de vin présentent une réduction de la surface de la crosse aortique par les dépôts lipidiques de 70 à 90% selon les molécules étudiées avec le même modèle de hamster sous régime athérogénique (Auger R et al. J. Agric. Food Chem. (2005) 53:9823 ; Auger R et al. (2005) J. Agric. Food Chem. 53:2015 ; Auger R et al. (2004) J. Agric. Food Chem. 52:5297).

Seul F4 augmente la capacité antioxydante du plasma, de 33,1% par rapport au groupe témoin. Concernant, les activités des enzymes hépatiques impliquées dans le système antioxydant, on observe que l'activité de la glutathion péroxydase séléno-dépendante est augmentée, et que l'activité SOD est fortement diminuée par les traitements avec F4 et F7 par rapport au groupe témoin.

On peut aussi constater une diminution très importante (60 à 70 %) de l'acide urique ainsi que de l'urée dans le plasma pour des deux groupes traités avec F4 et F7. Les réductions observées traduisent une élimination plus importante de l'acide urique et de l'urée par le rein ce qui diminue le risque d'hyperuricémie, paramètre est connu pour favoriser le développement de la plaque d'athérome.

L'amélioration importante du profil lipidique et des paramètres associés pour le modèle animal utilisé permet de conclure que la consommation régulière de chitine-glucane hydrolysé est bénéfique pour la prévention de l'athérosclérose et par extension sur l'obésité, avec des effets comparables, voire meilleurs que ceux induits par la consommation de chitosane.

**EXEMPLE 4- Effet de l'administration orale de chitine-glucane sur le tractus gastro-intestinal, le bilan glucidique et le bilan lipidique chez le rat**

**Protocole**

Le modèle utilisé est le rat sain sous alimentation standard et boisson enrichie en fructose (21%), qui favorise une stéatose hépatique, via une une métabolisation rapide du fructose par le foie résultant en une hypertriglycéridémie, une hyperinsulinémie et une diminution de l'action de l'insuline au niveau du muscle squelettique et du foie.

Le chitine-glucane (tableau 14) est administré dans la diète quotidienne des rats à raison de 10%, dose capable d'engendrer un effet aigu chez l'animal. Les effets à court terme et à long terme sont étudiés. Le groupe témoin reçoit une alimentation standard enrichie en fructose (Tableau 15). 10 rats mâles Wistar d'environ 100-125 g, sont répartis dans des cages en deux groupes (contrôle/traité) dans une pièce à 23°C, avec une hygrométrie de 70% et sous photopériode de 12 heures (12N/12D).

La consommation alimentaire et l'évolution du poids sont mesurées une fois par semaine. La production de fèces sur 24h ainsi que le contenu en eau de celles-ci sont estimés après 2 semaines de traitement. Après la période d'acclimatation (1 semaine) où les animaux reçoivent une alimentation standard non enrichie en fructose, une évaluation de la vidange gastrique et de la réponse glycémique en présence du chitine-glucan est effectuée. Les rats sont mis à jeun durant 18 heures. Le groupe traité reçoit une solution de glucose-paracétamol-chitine-glucane (10%) par gavage tandis que le groupe control reçoit une solution glucose-paracétamol. Des prises de sang sont effectuées durant 2 heures (glycémie, insulinémie, paracétamol). De même, une évaluation de la vidange gastrique et de la réponse glycémique en absence de chitine-glucane est effectuée après 3 semaines de traitement. Dans ce cas, les rats sont mis à jeun durant 18h et une solution de glucose-paracétamol est administrée par gavage durant 2 jours. Des prises de sang sont effectuées durant 2 heures (glycémie, insulinémie, paracétamol). Au cours de la troisième semaine de traitement, les rats sont mis à jeun durant 18 heures. Ensuite, une évaluation du temps de transit oro-fécal est effectuée. Les diètes colorées avec une solution de rouge Carmin sont présentées aux rats. Le temps d'apparition des premières fèces colorées est déterminé.

Après 4 semaines de traitement, les rats sont anesthésiés et une laparatomie est effectuée. Le sang des veines porte et cave est centrifugé afin de récupérer le sérum et/ou le plasma. Les bilans lipidique (cholestérol total, HDL-cholestérol, LDL-cholestérol) et glucidique (glucose, insuline) sont analysés à partir de ces derniers. Les différents organes sont prélevés, pesés et conservés afin d'estimer la fermentation caecal (prolifération caecal, pH caecal, contenu en acides carboxyliques à chaînes courtes), le contenu en lipide du foie et des fèces et, la masse adipeuse (poids des tissus adipeux épidydimale et viscérale).

**Résultats**

Les résultats montrent qu'une consommation régulière de chitine-glucane favorise un transit intestinal équilibré ainsi qu'un équilibre de l'homéostasie des bilans lipidique et glucidique dans un modèle de rat. On voit que l'« effet fibre » a un effet préventif sur les paramètres directement liés des maladies métaboliques telles que l'hypercholestérolémie, le diabète ou par extension le syndrome métabolique et l'obésité. En effet les fibres réduisent la densité calorique de la nourriture (nombre de calories pour 100 g), ralentissent significativement la vidange gastrique et la digestion des aliments, et réduisent significativement la sécrétion d'insuline. La conjonction de ces effets conduit à une diminution spontanée de la consommation calorique et de la sensation de faim.

Le chitine-glucane induit une fermentation au niveau caecal. Cette fermentation est accompagnée de la production d'acides gras à chaînes courtes significativement plus élevée dans le groupe recevant le chitine-glucane par rapport au groupe contrôle, en influençant l'homéostasie cellulaire épithéliale. Ceci contribue ainsi à réduire les risques d'atrophie, à stimuler la récupération d'un épithélium intestinal endommagé, et/ou à inhiber l'hyper-prolifération cellulaire.

**Tableau 14-** Caractéristiques moléculaires et composition du copolymère chitine-glucane

| Rapport chitine-glucane | Cendres | Protéines | Lipides | Métaux lourds |
|---|---|---|---|---|
| 35/65 | 2,3 | 7,71 | 1,5 | < 20 |

**Tableau 15-** Composition de l'alimentation des rats (diète AO4 standard, UAR, France)

| | % |
|---|---|
| Protéines | 19.3 |
| Glucides : | 70.4 |
|     - Cellulose | 5 |
|     - Amidon | 38 |
|     - Saccharose | 3 |
|     - Autres non digestibles | 8 |
| Lipides | 3 |

(suite)

| | % |
|---|---|
| Mélanges minéraux/vitamines | 7.3 |

**Tableau 16-** Variation des paramètres biochimiques et physiologiques des groupes de rats ayant consommé le chitine-glucane, par rapport au groupe contrôle

| | Chitine-glucane (CG) |
|---|---|
| Evolution du poids | ↘ |
| Consommation en nourriture | ↘ |
| glycémie | ≅ |
| insulinémie | ↘ |
| Masse adipeuse | ↘ |
| Production de fèces sur 24h | ↗ |
| Vidange gastrique | ↘ |
| Transit oro-fécal | ↘ |
| Fermentation caecale | ↗ |
| ↘ : significativement plus faible que la valeur du groupe contrôle ($p<0.05$) ; ↗ : significativement plus élevé que la valeur du groupe contrôle ($p<0.05$) ; ≅ significativement inchangé ($p<0.05$) | |

**EXEMPLE 5- Capacité d'absorption en eau du chitine-glucane**

Afin de mimer le comportement du chitine-glucane dans le tractus gastro-intestinal, la poudre de chitine-glucane est mise en présence d'eau, de NaCl et de solutions aqueuses à différents pH. Après un temps de contact de 12 heures sous agitation, on sépare le chitine-glucane par centrifugation, et on détermine la masse humide.

**Tableau 17-** Capacité de gonflement du chitine-glucane dans différents milieux (g d'eau absorbé par 100 g de chitine-glucane sec)

| Eau | NaCl 5% | pH 3 | pH 5 | pH 7 | pH 9 |
|---|---|---|---|---|---|
| 7 | 7 | 7 | 6 | 7 | 7 |

**Résultats**

On voit par cet exemple que le chitine-glucane est capable d'absorber environ 7 fois sa masse en eau ou en milieu aqueux, ce qui est du même ordre de grandeur que le taux gonflement des fibres alimentaires insolubles commerciales connues, comme par exemple l'hémicellulose et la pectine (4 à 8 fois leur masse en eau).

**EXEMPLE 6- Mise en évide de l'effet anti-antérosclérose, antioxydant, hypo-cholestérolémiant, hypolipidémiant après l'administration orale de chitine-glucane chez le hamster**

**Protocole**

Le modèle utilisé est le même que celui présenté dans l'Exemple 3. Dans cet exemple, deux composés dont les caractéristiques sont résumées dans le tableau 6.1. ont été administrés dans la diète quotidienne des hamsters :

- **Groupe CG2 :** 42.85 mg/kg/jour de chitine-glucane (soit 2 g/jour pour un homme de 70 kg)
- **Groupe CG1.5** : 21.43 mg/kg/jour de chitine-glucane (soit 1.5 g/jour pour un homme de 70 kg)
- **Groupe Cs2 :** 28.57 mg/kg/jour de chitosane (soit 2 g/jour pour un homme de 70 kg)

Le groupe témoin reçoit de l'eau par gavage journalier afin d'obtenir les mêmes conditions expérimentales.

**Tableau 18-** Caractéristiques des composés chitine-glucane et chitosane

|  | Chitine-glucane (CG) | Chitosane (Cs) |
|---|---|---|
| Masse moléculaire | N/A | 29,000 |
| Chitine (% du copolymère chitine-glucane) | 35 | N/A |
| Beta-glucane (mol% de la partie chitine) | 65 | 2.1 |
| Glucosamine (mol% de la partie chitine) | 0 | 89 |
| Cendres (%) | 2.3 | 2.66 |
| Protéines (%) | 7.7 | 0.31 |

Dès leur réception, 48 hamsters dorés syriens d'environ 100 g, sont placés dans les mêmes conditions expérimentales de l'exemple 3. La consommation alimentaire et leurs poids sont mesurés tous les jours. Les groupes reçoivent la même alimentation que celle présentée dans l'exemple 3 (Tableau 5).

**Résultats**

Les prélèvements, les analyses statistiques et les méthodes d'analyses sont les mêmes que celles présentées dans l'Exemple 3. L'ensemble des résultats est présenté dans le tableau 19.

**Tableau 19-** Variation des paramètres biochimiques des groupes de hamsters ayant consommé le chitine-glucane et le chitosane, par rapport au groupe contrôle

|  | Groupe CG2 (2 g/jour) | Groupe CG1.5 (1.5 g/jour) | Groupe Cs2 (2 g/jour) |
|---|---|---|---|
| Cholestérol total | ↘ | ↘ | ↘ |
| Triglycérides | ↘ | ↘ | ↘ |
| HDL-Cholestérol | ↗ | ↗ | ↗ |
| LDL-cholestérol | ↘ | ↘ | ↘ |
| Capacité antioxydante | ↗ | ↗ | ↘ |
| Acide urique | ↘ | ↘ | ↘ |
| Urée | ↘ | ↘ | ↘ |
| Se GSHPx | ↗ | ↗ | ↗ |
| SOD | ↘ | ↘ | ↘ |
| Pourcentage de dépôts lipidiques | ↘ | ↘ | ↘ |
| ↘ : significativement plus faible que la valeur du groupe contrôle ($p < 0.05$) ; ↗ : significativement plus élevé que la valeur du groupe contrôle ($p < 0.05$) | | | |

On voit par cet exemple que le chitine-glucane du entraîne les mêmes variations des paramètres biochimiques des rats sous régime athérogénique que les produits étudiés dans l'exemple 3. Le chitine-glucane améliore de manière importante le profil lipidique et les paramètres associé, aux deux doses étudiées. La consommation régulière de chitine-glucane est donc bénéfique dans la prévention de l'athérosclérose, et par extension des pathologies associées.

**Revendications**

**1.** Polysaccharide d'origine fongique ou extrait fongique comprenant majoritairement un copolymère chitine-glucane, ou composition comprenant un tel polysaccharide, pour son utilisation dans la prévention, le traitement, ou la lutte contre la dyslipidémie, l'hypercholestérolémie, l'athérosclérose, une pathologie associée à l'athérosclérose, l'obésité, une maladie cardiovasculaire, le syndrome métabolique, le diabète, l'hyperglycémie ou l'hyperuricémie, ou pour favoriser la satiété, favoriser le transit alimentaire, ou favoriser l'équilibre de l'homéostasie lipidique et glucidique , ladite utilisation comprenant l'administration par voie orale dudit polysaccharide d'origine fongique ou de ladite composition le comprenant, à un être humain ou un animal, de préférence un mammifère.

2. Polysaccharide d'origine fongique, ou extrait fongique comprenant majoritairement un copolymère chitine-glucane, ou composition comprenant un tel polysaccharide, pour son utilisation selon la revendication 1, **caractérisé en ce que** le rapport entre la chitine et les beta-glucanes est compris entre 70:30 et 20:80.

3. Polysaccharide d'origine fongique, ou extrait fongique comprenant majoritairement un copolymère chitine-glucane, ou composition comprenant un tel polysaccharide, pour son utilisation selon la revendication 1 ou 2, **caractérisé en ce que** le rapport entre la chitine et les beta-glucanes est compris entre 50:50 et 10:90.

4. Polysaccharide d'origine fongique, ou extrait fongique comprenant majoritairement un copolymère chitine-glucane, ou composition comprenant un tel polysaccharide, pour son utilisation selon l'une quelconque des revendications 1 à 3, **caractérisé en ce que** le polysaccharide ou extrait fongique comprend plus de 70 % de polysaccharides chitine-glucane en masse par rapport à la masse totale de l'extrait d'origine fongique ou du polysaccharide.

5. Polysaccharide d'origine fongique, ou extrait fongique comprenant majoritairement un copolymère chitine-glucane, ou composition comprenant un tel polysaccharide, pour son utilisation selon l'une quelconque des revendications 1 à 4, **caractérisé en ce que** le polysaccharide ou extrait fongique comprend plus de 85 % de polysaccharides chitine-glucane en masse par rapport à la masse totale de l'extrait d'origine fongique.

6. Polysaccharide d'origine fongique, ou extrait fongique comprenant majoritairement un copolymère chitine-glucane, ou composition comprenant un tel polysaccharide, pour son utilisation selon l'une quelconque des revendications 1 à 5, **caractérisé en ce qu'**il s'agit d'un polysaccharide d'origine *Aspergillus niger* ou un extrait d'*Aspergillus niger.*

7. Polysaccharide d'origine fongique, ou extrait fongique comprenant majoritairement un copolymère chitine-glucane, ou composition comprenant un tel polysaccharide, pour son utilisation selon l'une quelconque des revendications 1 à 6, **caractérisé en ce qu'**il s'agit d'un hydrolysat du polysaccharide.

8. Polysaccharide d'origine fongique, ou extrait fongique comprenant majoritairement un copolymère chitine-glucane, ou composition comprenant un tel polysaccharide, pour son utilisation selon l'une quelconque des revendications 1 à 6, **caractérisé en ce qu'**il s'agit d'un hydrolysat de chitine-glucane ayant un rapport entre la chitine et les beta-glucanes compris entre 60:40 et 15:85 (m/m).

9. Polysaccharide d'origine fongique, ou extrait fongique comprenant majoritairement un copolymère chitine-glucane, ou composition comprenant un tel polysaccharide, tel que défini selon l'une quelconque des revendications 1 à 8, pour son utilisation comme composition pharmaceutique dans la prévention, le traitement, ou la lutte contre une dyslipidémie et/ou une hypercholestérolémie.

10. Polysaccharide d'origine fongique, ou extrait fongique comprenant majoritairement un copolymère chitine-glucane, ou composition comprenant un tel polysaccharide, tel que défini selon l'une quelconque des revendications 1 à 8, pour son utilisation comme composition pharmaceutique pour favoriser la satiété et/ou le transit alimentaire d'un être humain ou animal.

11. Polysaccharide d'origine fongique, ou extrait fongique comprenant majoritairement un copolymère chitine-glucane, ou composition comprenant un tel polysaccharide, tel que défini selon l'une quelconque des revendications 1 à 8, pour son utilisation comme composition pharmaceutique dans la prévention, le traitement, ou la lutte contre une maladie cardiovasculaire.

12. Utilisation non thérapeutique d'une composition comme complément alimentaire pour favoriser la satiété ou le transit alimentaire d'un être humain ou animal, ladite composition comprenant au moins un polysaccharide d'origine fongique ou au moins un extrait fongique comprenant majoritairement un copolymère chitine-glucane tel que défini selon l'une quelconque des revendications 1 à 8.

13. Utilisation d'au moins un polysaccharide d'origine fongique, ledit polysaccharide comprenant majoritairement un copolymère chitine-glucane, pour la fabrication d'une composition administrée par voie orale à un être humain ou un animal, de préférence un mammifère, pour la prévention, le traitement, ou la lutte contre une dyslipidémie, une hypercholestérolémie, l'athérosclérose, une maladie cardiovasculaire, le syndrome métabolique, le diabète, une hyperglycémie ou l'hyperuricémie, ou pour favoriser la satiété, favoriser le transit alimentaire, ou favoriser l'équilibre de l'homéostasie lipidique et glucidique .

14. Méthode de soin esthétique pour diminuer, prévenir ou lutter contre la prise de masse d'un être humain ou animal, et de préférence d'un mammifère, ladite méthode comprenant l'administration par voie orale d'un polysaccharide d'origine fongique ou extrait fongique comprenant majoritairement un copolymère chitine-glucane, ou d'une composition comprenant un tel polysaccharide, à un être humain ou un animal, de préférence un mammifère d'un polysaccharide d'origine fongique ou extrait fongique comprenant majoritairement un copolymère chitine-glucane.


## Patentansprüche

1. Von einem Pilz stammendes Polysaccharid oder Pilz-Extrakt, aufweisend mehrheitlich ein Chitin-Glukan-Copolymer, oder eine ein solches Polysaccharid aufweisende Zusammensetzung zu deren Verwendung in der Prävention, der Behandlung oder der Bekämpfung der Dyslipidämie, der Hypercholesterinämie, der Atherosklerose, einer mit der Atherosklerose assoziierten Pathologie, der Fettleibigkeit, einer kardiovaskulären Erkrankung, des metabolischen Syndroms, der Diabetes, der Hyperglykämie oder der Hyperurikämie oder zum Begünstigen des Sättigungsgefühls, Begünstigen der Nahrungspassage oder Begünstigen des homöostatischen Lipid- und Kohlenhydrat-Gleichgewichts, wobei die genannte Verwendung die Verabreichung des genannten von einem Pilz stammenden Polysaccharids oder der genannten dieses aufweisenden Zusammensetzung, auf oralem Wege an einen Menschen oder ein Tier, vorzugsweise ein Säugetier, aufweist.

2. Von einem Pilz stammendes Polysaccharid oder Pilz-Extrakt, mehrheitlich aufweisend ein Chitin-Glukan-Copolymer aufweisen, oder eine ein solches Polysaccharid aufweisende Zusammensetzung zu deren Verwendung gemäß Anspruch 1, **dadurch gekennzeichnet, dass** das Verhältnis des Chitins zu den Beta-Glukanen zwischen einschließlich 70:30 und 20:80 liegt.

3. Von einem Pilz stammendes Polysaccharid oder Pilz-Extrakt, die mehrheitlich ein Chitin-Glukan-Copolymer aufweisen, oder eine ein solches Polysaccharid aufweisende Zusammensetzung zu deren Verwendung gemäß Anspruch 1 oder 2, dadurch charakterisiert, dass das Verhältnis des Chitins zu den Beta-Glukanen zwischen einschließlich 50:50 und 10:90 liegt.

4. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung zu deren Verwendung gemäß irgendeinem der Ansprüche 1 bis 3, **dadurch gekennzeichnet, dass** das Polysaccharid oder der Pilz-Extrakt mehr als 70 Masse-% von Chitin-Glukan-Polysacchariden im Verhältnis zu der Gesamtmasse des Pilz-Extrakts oder des Polysaccharids aufweist.

5. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung zu deren Verwendung gemäß irgendeinem der Ansprüche 1 bis 4, **dadurch gekennzeichnet, dass** das Polysaccharid oder der Pilz-Extrakt mehr als 85 Masse-% von Chitin-Glukan-Polysacchariden im Verhältnis zu der Gesamtmasse des Pilz-Extrakts oder des Polysaccharids aufweist.

6. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung zu deren Verwendung gemäß irgendeinem der Ansprüche 1 bis 5, **dadurch gekennzeichnet, dass** es sich um ein von *Aspergillus niger* stammendes Polysaccharid oder ein Extrakt von *Aspergillus niger* handelt.

7. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung zu deren Verwendung gemäß irgendeinem der Ansprüche 1 bis 6, **dadurch gekennzeichnet, dass** es sich um ein Hydrolysat des Polysaccharids handelt.

8. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung zu deren Verwendung gemäß irgendeinem der Ansprüche 1 bis 6, **dadurch gekennzeichnet, dass** es sich um ein Hydrolysat von Chitin-Glukan handelt, das ein Verhältnis des Chitins zu den Beta-Glukanen zwischen einschließlich 60:40 und 15:85 (m/m) aufweist.

9. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung gemäß irgendeinem der Ansprüche 1 bis 8 zu deren Verwendung als pharmazeutische Zusammensetzung bei der Prävention, der Behandlung oder der Bekämpfung einer Fettleibigkeit und/oder eine Hypercholesterinämie.

10. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung gemäß irgendeinem der Ansprüche 1 bis 8, zu deren Verwendung als pharmazeutische Zusammensetzung zum Begünstigen des Sättigungsgefühls und/oder der Nahrungspassage eines Menschen oder Tieres.

11. Von einem Pilz stammendes Polysaccharid oder Pilz-Extrakt, mehrheitlich aufweisend ein Chitin-Glukan-Copolymer, oder eine ein solches Polysaccharid aufweisende Zusammensetzung gemäß irgendeinem der Ansprüche 1 bis 8 zu deren Verwendung als pharmazeutische Zusammensetzung bei der Prävention, der Behandlung oder der Bekämpfung einer kardiovaskulären Erkrankung.

12. Nicht-therapeutische Verwendung einer Zusammensetzung als Nahrungsergänzungsmittel zum Begünstigen des Sättigungsgefühls oder der Nahrungspassage eines Menschen oder Tieres, wobei die genannte Zusammensetzung mindestens ein von einem Pilz stammenden Polysaccharid oder mindestens ein Pilz-Extrakts aufweist, mehrheitlich aufweisend ein Chitin-Glukan-Copolymer, gemäß irgendeinem der Ansprüche 1 bis 8.

13. Verwendung mindestens eines von einem Pilz stammenden Polysaccharids, wobei das genannte Polysaccharid mehrheitlich ein Chitin-Glukan-Copolymer aufweist, zur Herstellung einer auf oralem Wege an einen Menschen oder ein Tier, vorzugsweise ein Säugetier, für die Prävention, die Behandlung oder die Bekämpfung einer Fettstoffwechselstörung, einer Hypercholesterinämie, der Atherosklerose, einer kardiovaskulären Erkrankung, des metabolischen Syndroms, der Diabetes, einer Hyperglykämie oder der Hyperurikämie oder zum Begünstigen des Sättigungsgefühls, Begünstigen der Nahrungspassage oder Begünstigen des homöostatischen Lipid- oder Kohlenhydrat-Gleichgewichts verabreichten Zusammensetzung.

14. Verfahren zur Schönheitsbehandlung zum Vermindern, Verhindern oder Bekämpfen der Gewichtszunahme eines Menschen oder Tieres, und vorzugsweise eines Säugetiers, wobei das genannte Verfahren die Verabreichung eines von einem Pilz stammenden Polysaccharids oder Pilz-Extrakts, mehrheitlich aufweisend ein Chitin-Glukan-Copolymer, oder einer ein solches Polysaccharid aufweisenden Zusammensetzung an einen Menschen oder ein Tier, vorzugsweise ein Säugetier, auf oralem Wege aufweist eines von einem Pilz stammenden Polysaccharids oder Pilz-Extrakts, die mehrheitlich ein Chitin-Glukan-Copolymer aufweisen.

**Claims**

1. Polysaccharide of fungal origin or fungal extract comprising mostly a chitin-glucan copolymer, or composition comprising such a polysaccharide, for its use in preventing, treating, or fighting against dyslipidemia, hypercholesterolemia, atherosclerosis, a pathological condition associated with atherosclerosis, obesity, cardiovascular disease, metabolic syndrome, diabetes, hyperglycemia or hyperuricemia, or for promoting satiety, promoting alimentary transit, or promoting stable lipid and glucose homeostasis, said use comprising the oral administration of said polysaccharide of fungal origin, or of said composition comprising it, to a human being or an animal, preferably a mammal.

2. Polysaccharide of fungal origin or fungal extract comprising mostly a chitin-glucan copolymer, or composition comprising such a polysaccharide, for its use according to claim 1, **characterized in that** the ratio of the chitin to the beta-glucans is between 70:30 and 20:80.

3. Polysaccharide of fungal origin or fungal extract comprising mostly a chitin-glucan copolymer, or composition comprising such a polysaccharide, for its use according to claim 1 or claim 2, **characterized in that** the ratio of the chitin to the beta-glucans is between 50:50 and 10:90.

4. Polysaccharide of fungal origin or fungal extract comprising mostly a chitin-glucan copolymer, or composition comprising such a polysaccharide, for its use according to any one of claims 1 to 3, **characterized in that** the polysaccharide or fungal extract comprises more than 70% by weight of chitin-glucan polysaccharides in proportion to the total weight of the extract of fungal origin or of the polysaccharide.

5. Polysaccharide of fungal origin or fungal extract comprising mostly a chitin-glucan copolymer, or composition comprising such a polysaccharide, for its use according to any one of claims 1 to 4, **characterized in that** the polysaccharide or fungal extract comprises more than 85% by weight of chitin-glucan polysaccharides in proportion to the total weight of the extract of fungal origin.

6. Polysaccharide of fungal origin or fungal extract comprising mostly a chitin-glucan copolymer, or composition comprising such a polysaccharide, for its use according to any one of claims 1 to 5, **characterized in that** it is a polysaccharide of *Aspergillus niger* origin or an extract of *Aspergillus niger.*

7. Polysaccharide of fungal origin or fungal extract comprising mostly a chitin-glucan copolymer, or composition comprising such a polysaccharide, for its use according to any one of claims 1 to 6, **characterized in that** it is a hydrolysate of the polysaccharide.

8. Polysaccharide of fungal origin or fungal extract comprising mostly a chitin-glucan copolymer, or composition comprising such a polysaccharide, for its use according to any one of claims 1 to 6, **characterized in that** it is a chitin-glucan hydrolysate having a chitin to beta-glucan ratio between 60:40 and 15:85 (m/m).

9. Polysaccharide of fungal origin or fungal extract comprising mostly a chitin-glucan copolymer, or composition comprising such a polysaccharide, as defined according to any one of claims 1 to 8, for its use as a pharmaceutical composition in preventing, treating, or fighting against dyslipidemia and/or hypercholesterolemia.

10. Polysaccharide of fungal origin or fungal extract comprising mostly a chitin-glucan copolymer, or composition comprising such a polysaccharide, as defined according to any one of claims 1 to 8, for its use as a pharmaceutical composition for promoting satiety and/or alimentary transit in a human being or animal.

11. Polysaccharide of fungal origin or fungal extract comprising mostly a chitin-glucan copolymer, or composition comprising such a polysaccharide, as defined according to any one of claims 1 to 8, for its use as a pharmaceutical composition in preventing, treating, or fighting against cardiovascular disease.

12. Non-therapeutic use of a composition as a dietary supplement for promoting satiety or alimentary transit in a human being or animal, said composition comprising at least one polysaccharide of fungal origin or at least one fungal extract comprising mostly a chitin-glucan copolymer as defined according to any one of claims 1 to 8.

13. Use of at least one polysaccharide of fungal origin, said polysaccharide comprising mostly a chitin-glucan copolymer, for manufacturing a composition administered orally to a human being or to an animal, preferably a mammal, for preventing, treating, or fighting against dyslipidemia, hypercholesterolemia, atherosclerosis, cardiovascular disease, metabolic syndrome, diabetes, hyperglycemia or hyperuricemia, or for promoting satiety, promoting alimentary transit, or promoting stable lipid and glucose homeostasis.

14. Esthetic care method for reducing, preventing, or fighting against weight gain in a human being or animal, and preferably in a mammal, said method comprising the oral administration of a polysaccharide of fungal origin or fungal extract comprising mostly a chitin-glucan copolymer, or of a composition comprising such a polysaccharide, to a human being or an animal, preferably a mammal, of a polysaccharide of fungal origin or fungal extract comprising mostly a chitin-glucan copolymer.

```
Current Data Parameters
NAME            JGgautier
EXPNO                 106
PROCNO                  1

F2 - Acquisition Parameters
Date_            20040713
Time_              10.15
INSTRUM             spect
PROBHD   BL4    N-P/B 40
PULPROG          cp4c.98
TD                  3800
SOLVENT
NS                   600
DS                     0
SWH          39920.160 Hz
FIDRES       10.505305 Hz
AQ            0.0476450 sec
RG               262144
DW              12.525 use
DE              14.29 use
TE              300.0 K
D1          5.00000000 sec

============== CHANNEL f1 ===
NUC1                 13C
P15            2500.00 use
PL1              17.50 dB
SFO1        100.6353010 MHz

============== CHANNEL f2 ===
CPDPRG2           tppm15
NUC2                 1H
P3               3.00 use
P31              5.80 use
PL2              9.00 dB
PL12             7.50 dB
SFO2        400.1720000 MHz
SPO              10.50 dB
SPNAM0         square.64
SPOFF0           0.00 Hz

F2 - Processing parameters
SI                  8192
SF          100.6228351 MHz
WDW                   EM
SSB                    0
LB               40.00 Hz
GB                     0
PC                  0.50

-1D NMR plot parameters
CX               20.00 cm
F1P             120.000 ppm
F1            12074.74 Hz
F2P              0.000 ppm
F2               0.00 Hz
PPMCM          6.00000 ppm
HZCM          603.73700 Hz/
```

FIG.1

20

LCB 28-4, rot 7kHz, 11:14 AM 9/1/2005

FIG.2

**FBI-33, rot 7kHz, 10:32 AM 7/13/2004**

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0135944 A
- WO 03068824 A

- FR 0507066

**Littérature non-brevet citée dans la description**

- **FUKUSHIMA M ; NAKANO M ; MORII Y ; OHASHI T ; FUJIWARA Y ; SONOYAMA K.** *J. Nutr.,* 2000, vol. 130, 2151
- **LULL C ; WICHERS HJ ; SAVELKOUL HFJ.** Anti-inflammatory and immunomodulating properties of fungal metabolites. *Mediators Inflammation,* 2005, vol. 2, 63
- **SIESTMA JH ; WESSELS JG.** Solubility of (1,3)-beta-D-(1,6)-beta-D-glucan in fungal walls : importance of presumed linkage between glucan and chitin. *J. Gen. Microbiol.,* 1981, vol. 125, 209
- **STAGG CM ; FEATHER MS.** *Biochim. Biophys.,* 1973, vol. 320, 64
- **FONTAINE T ; SIMENEL C ; DUBREUCQ G ; ADAM O ; DELEPIERRE M ; LEMOINE J ; VORGIAS CE ; DIAQUIN M ; LATGÉ JP.** Molecular organization of the alkali-insoluble fraction of Aspergillus fumigatus cell wall. *J. Bio. Chem.,* 2000, vol. 275, 27594
- **KOLLAR R ; PETRAKOVAS E ; ASHWELL G ; ROBBINS P ; CABIB E.** Architecture of the yeast cell wall, the linkage between chitin and beta(1,3)glucan. *J. Biol. Chem.,* 1995, vol. 270, 1170
- **HEUX L ; BRUGNEROTTO J ; DESBRIÈRES J ; VERSALI MF ; RINAUDO M.** Solid state NMR for determination of the degree of acetylation of chitin and chitosan. *Biomacromolecules,* 2000, vol. 1, 746
- **NISTOR A ; BULLA A ; FILIP DA ; RADU A.** The hyperlipidemic hamster as a model of experimental atherosclerosis. *Atherosclerosis,* 1987, vol. 68, 159
- **KOWALA, MC ; NUNNARI, JJ ; DURHAM, SK ; NICOLOSI, RJ.** Doxazosin and cholestyramine similarly decrease fatty streak formation in the aortic arch of hyperlipidemic hamsters. *Atherosclerosis,* 1991, vol. 91, 3549

- **NUNNARI JJ ; ZAND T ; JORIS I ; MAJNO G.** Quantification of oil Red O staining of the aorta in hypercholesterolemic rats. *Exp. Mol. Pathol.,* 1989, vol. 51, 1
- **ALLAIN CC ; POON LS ; CHAN CS ; RICHMOND W ; FU PC.** Enzymatic determination of total serum cholesterol. *Clin. Chem.,* 1974, vol. 20, 470
- **WAHLEFELD AW ; BERGMEYER HU.** Methods of enzymatic analysis Second Edition. Academic Press, 1974, 1831
- **MÂRZ W ; GROSS W.** Evaluation of a phosphotungstic acid/MgCl2 precipitation and quantitative lipoprotein electrophoresis assay. *Clin. Chim. Acta.,* 1986, vol. 158, 33
- **TALKE H ; SCHUBERT GE.** Enzymatische Harnstoffbest timmung im blut und serum im optischen test nach Warburg. *Klin. Wochenschr,* 1965, vol. 43, 174
- **TOWN MH et al.** *J. Clin. Chem. Clin. Biochem.,* 1985, vol. 23, 591
- **MILLER NJ ; RICE-EVANS C ; DAVIES MJ ; GOPINATHAN V ; MILNER A.** A novel method for measuring antioxydant capacity and its application to monitoring the antioxydant status in premature neonates. *Clinical Science,* 1993, vol. 84, 407
- **SMITH PK et al.** Measurement of protein using bicinchoninic acid. *Anal. Biochem.,* 1985, vol. 150, 76
- **WENDEL A.** Glutathione peroxydase. *Methods in enzymology,* 1981, vol. 77, 327
- **PAOLETTI F ; MOCALI A.** Determination of superoxyde dismutase activity by purely chemical system based on NADCPJH oxidation. *Methods Enzymology,* 1990, vol. 186, 209
- **AUGER R et al.** *J. Agric. Food Chem.,* 2005, vol. 53, 9823
- **AUGER R et al.** *J. Agric. Food Chem.,* 2005, vol. 53, 2015
- **AUGER R et al.** *J. Agric. Food Chem.,* 2004, vol. 52, 5297